**Europäisches Patentamt**

**European Patent Office**

**Office européen des brevets**

(19)

(11) Publication number : **0 485 663 B1**

# EUROPEAN PATENT SPECIFICATION

(12)

(45) Date of publication of patent specification :
**20.10.93 Bulletin 93/42**

(51) Int. Cl.[5] : **A23J 3/08,** A23J 1/20,
A23D 7/00, A23L 1/24,
A61K 7/48

(21) Application number : **90202991.7**

(22) Date of filing : **12.11.90**

(54) **Edible composition of denatured whey proteins.**

The file contains technical information
submitted after the application was filed and
not included in this specification

(43) Date of publication of application :
**20.05.92 Bulletin 92/21**

(45) Publication of the grant of the patent :
**20.10.93 Bulletin 93/42**

(84) Designated Contracting States :
**AT BE CH DE DK ES FR GB GR IT LI NL SE**

(56) References cited :
**EP-A- 0 250 623**
**EP-A- 0 347 237**
**EP-A- 0 412 590**
**WO-A-88/08673**

(73) Proprietor : **QUEST INTERNATIONAL B.V.**
**Huizerstraatweg 28**
**NL-1411 GP Naarden (NL)**

(72) Inventor : **Hakkaart, Marcellinus Jacobus J.**
**Surinamelaan 18**
**1213 VN Hilversum (NL)**
Inventor : **Kunst, Anthonie**
**Weegbree 41**
**1273 AN, Huizen (NL)**
Inventor : **Leclercq, Edith**
**de Vecht 26**
**3448 CM, Woerden (NL)**
Inventor : **de Levita, Paul David**
**Buzziburglaan 16**
**3972 EE, Driebergen (NL)**
Inventor : **Moonen, Johannes Hubertus Elise**
**Esther de Boer van Rijklaan 23**
**3584 GL, Utrecht (NL)**

(74) Representative : **Mulder, Cornelis Willem**
**Reinier, Dr. et al**
**UNILEVER N.V. Patent Division P.O. Box 137**
**NL-3130 AC Vlaardingen (NL)**

EP 0 485 663 B1

## Description

The present invention is concerned with a process for the preparation of a food composition comprising colloidal particles of at least partly denatured protein with a fatty mouthfeel impression, with aqueous compositions comprising such colloidal particles, with dry compositions obtainable by drying such aqueous compositions, and with food products and topical compositions, containing these compositions.

In European patent application 0 250 623 an aqueous dispersion is described comprising macro-colloidal non-aggregated particles of denatured whey protein coagulate with mean diameter particle size distribution when dry ranging from about 0.1 to about 2.0 micrometer (microns). The dispersions are prepared by heating an aqueous concentrate of undenatured whey protein at heat denaturing temperatures from about 80°C to 130°C, in an aqueous solution under very high shear conditions at pH below the isoelectric point. In European patent application 0 323 529 a similar process is described, starting from other types of protein than whey protein.

A comparable edible product comprising an aqueous macrocolloidal dispersion can be obtained without the necessity of subjecting the aqueous protein solution to high shear condition, by a process comprising successively heating (60 - 80°C) and concentrating (European patent application 0 347 237). The heating is carried out at pH=6.5-7.0. The mixture is cooled to about 35°C and the pH is adjusted to about 4.6.

We have now found that the alpha-lactalbumins, present in protein compositions can be denatured selectively and be separated from the other protein present. In this way a product enriched in alpha-lactalbumin can be obtained, that possesses an excellent unctuous fatty mouthfeel, and texturising properties.

In literature various processes are described for separating and isolating various whey protein fractions, mainly alpha-lactalbumin and beta-globulin. De Wit, J.N., Klarenbeek, G. & Adamse, M. (Neth. Milk Dairy J., 40, no. 1, pp. 41-56 (1986) have compared various processes for isolating whey proteins. Among them are membrane processes such as ultrafiltration and diafiltration, ionic exchange processes based on resins from Sphérosil or Vistec, and whey desalting and desugaring processes as used by lactose manufacturers. Other processes for separating and isolating whey proteins are heat treatment (65°C) at pH 4.1-4.3 for several minutes (Pearce, R.J., Austr. J. Dairy Technol., vol. 38, pp. 144-148 (1983); precipitation of beta-lactoglobulin at pH 4.65 (Slack, A.W., Amundson, C.H. & Hill, C.G., J. Food Proc. Preserv., vol. 10, pp 19-30 (1986); salting out of alpha-lactalbumin at low pH (Mailliart, P. & Ribadeau-Dumas, B., J. Food Sci., vol 54, 743-745, 752 (1988).

All processes mentioned above are fractionating whey proteins for functionality and nutritional purposes. Especially beta-lactoglobulin is referred to as having emulsifying and whipping properties, and is mostly used in fortified diet specialty products, whereas alpha-lactalbumin constitute a waste.

The present invention describes the isolation of an enriched fraction of denatured alpha-lactalbumin via heat treatment at neutral pH according to European patent application 0 347 237, which due to specific pH setting and cooling regime after heating gives an excellent unctuous fatty mouthfeel, and has a consistency similar to spreads, and which can be used as texturising agent in emulsion type food and cosmetic products, like ice cream, dressing, cheese, yoghurt, spread, cream, lotion, when applied as wet fraction, and can act as emulsifier in food and cosmetic products when applied as protein powder.

Normally protein fractions of cheese whey consist for about 19-21% of alpha-lactalbumin, and for about 51-54% of β-lactoglobulin so the weight ratio between these two varies from 0.35 to 0.41.

HPLC pictures of the protein of the obtained edible composition, and of the solution of undenatured protein, show that in the edible protein composition obtained after separation the weight ratio α-lactalbumin to β-lactoglobuline based upon the protein present in the whey is increased. Therefore according to the invention a fractionation of whey proteins in a denatured alpha-lactalbumin enriched fraction (sludge) and an undenatured beta-lactoglobulin enriched fraction is obtained.

Thus the invention concerns with a process for the preparation of a food composition, the process comprising successively heating of an aqueous composition at 60-80°C and at a pH of 6.0-7.2 comprising less than 15% by weight of the water, of heat denaturable protein at least partly consisting of whey protein, to at least partly denature said protein, cooling of the composition to a temperature of not less than 45°C, adjusting the pH of the composition to 4.2-5.0 and removing at least part of the remaining undenatured protein present, to provide a composition of at least 5 wt.% of heat denatured protein in the form of microcolloidal particles having a mean size in the range of 0.1 to 10 micrometer (microns) at pH= 4.2-5.0, in particular 3.0-5.0 um, in which the alpha-lactalbumin content and the β-lactoglobulin content, based upon the protein present in the whey are such that the weight-ratio of α-lactalbumin to β-lactoglobuline is more than 0.43. It is emphasized here that Microtrac that is used as measuring technique does not detect particles with sizes below 0.1 μm.

In a preferred embodiment the removal of undenatured protein is carried out in such a way that the weight ratio of denatured alpha-lactalbumin to β-lactoglobulin in the composition based upon the protein present in the whey is 0.55 - 4.0.

In the present process the heat-denaturable protein applied, is a delactosed demineralised protein, preferably ultrafiltered whey protein.

Although in the present process the aqueous composition, prior to the heat treatment may contain as much as 15%, by weight of the water, of heat denaturable protein, it is preferred to apply a protein content of less than 8 wt.% and more than 0.5 wt.% of heat denaturable protein. If relatively low concentration levels are applied the final products appear to have very smooth but less fatty characteristics.

In a preferred embodiment of the present process the aqueous composition is heated in the heating step for a period from 1 to 30 minutes to a temperature of 60°C-75°C. Although it may be desirable to stir the aqueous composition, no benefits are obtained from subjecting said composition to high shear.

It was found that during this mild process no off-flavours are generated. The development of such off-flavours is undesirable if the present process is used for the preparation of ingredients for food products or farmaceuticals or cosmetic products.

After the heating the mixture obtained is cooled to a temperature of not less than 45°C. This cooling is essential in order to obtain products with the required characteristics. If the cooling is performed to lower temperatures, the product is loosing part of its texturising characteristics.

After the cool treatment the pH is adjusted to pH= 4.2-5.0, where upon the aqueous composition may suitable be concentrated by the application of any concentrating technique known in the art. Examples of such techniques are: membrane filtration, ion exchange, or more preferably centrifugation. The suitability of these techniques depend on the ingredients present in the aqueous composition and on the desired degree of concentration in addition to effectiveness in removing undenatured whey protein.

The concentrating step in the present process is required to remove part of the undenatured protein and attain the desired consistency. The more the aqueous composition is concentrated, i.e. the water content reduced, the more firm the concentrated product becomes. Thus, in practice, the degree of concentrating required merely depends on the consistency desired. In order to produce compositions having an unctuous fatty mouthfeel, and a spreadable consistency, it is necessary to concentrate the aqueous composition to a protein content of at least 7 wt.%, more preferably to a protein content of between 8 and 30 wt.%.

The aqueous composition may alternatively be dried, for example by freeze drying or spray drying. However, the concentrated product has to be diluted with water or a carbohydrate solution when spraydried because of the high viscosity of the concentrated product. Mono- as well as di- and oligosaccharides, most preferably lactose or saccharose, may be used as filling material during spraydrying in a concentration ratio protein: carbohydrate up to 1 : 20, preferably 1 : 1. The ratio between protein and carbohydrate during freezing and freeze drying is the same. Addition of a carbohydrate during drying of protein composition gives a stable powder, and a good reconstitutable and rehydratable product with the same characteristics as the original, not dried protein composition. This possibility of drying and rehydration of the product means a main advantage over the products that are described in US 4,734,287. In column 14, lines 38-64 of this US patent it is set out that the products are difficult to rehydrate because of glueing.

The composition obtained by means of the present process comprises protein-based particles having a mean size, as determined by a Microtrac Small Particle Analyzer (Tradename) in the range of 0.1 to 10 micrometer (microns) (at pH: 4.2-5.0). If the diameter of the particles becomes rather large, deficiencies such as graininess and imperfection of fatty mouthfeel are observed. Particle size measurements according to the technique, described in column 22, lines 31-50 in Monsanto's US patent 4,734,287 were not possible, as the product dissolved during the neutralisation step, which is imperative in this method.

The fatty mouthfeel of the present protein composition may be improved by addition of emulsifiers, stabilizers, and thickeners, during the preparation of the said protein composition. Addition of the above mentioned compounds may occur before the heating step, after the heating step, before acidification, after acidification, or mixed with the already prepared protein composition. Most preferably the emulsifiers, stabilizers or thickeners are added to the protein solution prior to heating. As additives may be used all types of emulsifiers, most preferably emulsifiers with rather low HLB-value, e.g. HLB-value not above 10, most preferably HLB-value lower than 8. Examples of emulsifiers are Spans (sorbitan esters), mono- and diglycerides, lecithin and egg yolk. Concentration of emulsifier used may be up to 5.0 wt.%, preferably 0.1-0.3 wt.%. Thus the weight ratio of protein to emulsifier may be above 1:1, preferably in the range of 50:1 to 15:1.

Examples of stabilizers and thickeners are starch (optionally modified), gums, gelatin, pectin, cellulose (optionally modified), casein. Concentration of the stabilizers and thickeners used may be up to 10.0 wt.%, preferably 0.5-3.0%. The weight ratio of protein to stabilizer or thickener may be up to 1:2, preferably in the range of 10:1 to 2:1.

The presence of molecules such as emulsifiers, stabilisers and organic acids (e.g. citric acid, lactic acid, phosphoric acid) and minerals in the product can have an effect on the flavour of the endproduct application and need to be carefully chosen per application.

3

The wet products of the invention exhibit good microbiological stability and if stored with a preservative, e.g. potassium sorbate at 5°C for over three months without significant deterioration. Addition of a preservative seems not always necessary since protein composition may be pasteurized, optionally prior to concentration. The dried product may be stored at room temperature, with or without a preservative e.g. potassium sorbate, for at least one year without significant deterioration. They can be used to provide a low calorie food product or cosmetic product. This way margarine, dressing, cheese, yoghurt, frozen dessert such as ice cream, mayonnaise, dairy and non-dairy creams can be made. In most applications, especially in products with a pH below 6.0, the product of the current invention will give a perceived "melting-down" of the product in the mouth, resembling melting of gelatin or fat emulsion systems. This effect can be explained by the relative instability of the product of the present invention especially at neutral pH, a pH as present also in the salivary system in the mouth: the product starts to decompose (resolubilize) at these pH conditions. The product can be optimised and tailored in this respect for each application. It is anticipated that after consumption of the food product and partial decomposition of the product of the present invention, the product arrives in the stomach, where it can reprecipitate/reflocculate at the pH conditions of the stomach and generate a feeling of satiety for a period of time before hydrolysis of the protein. The products may also be incorporated in creams and other products for topical application to the body, for example in cosmetics, optionally in combination with an acceptable support material, for example a fat-based cream, for obtaining a low-fat edible cosmetic cream.

The present invention is illustrated by the following examples:

### Example 1

1 litre of 5% aqueous solution (pH 7.2) of Bipro (R-Tradename) undenatured delactosed, demineralised whey protein powder (ex. Bio-isolates PLC, Swansea, UK), was partly denatured by heating 10 minutes at 65°C. This colloidal stable, colloidal dispersion was thus obtained. The dispersion was cooled to 50°C and acidified to pH 4.8 with 20 wt.% citric acid solution. The dispersion was concentrated by centrifuge at 3000 rpm after which a spreadable mass, containing about 12 wt.% of mainly alpha-lactalbumin and 88 wt.% of water with undenatured protein was obtained.

HPLC pictures of the protein solution before heating, of the obtained edible composition, and of the solution of, undenatured protein, show that about 75 wt.% of denatured alpha-lactalbumin is present in the edible protein composition, thus this process is fractionating whey proteins in a alpha-lactalbumin enriched fraction (sludge) and a beta-lactoglobulin enriched fraction, undenatured in solution.

The distribution of the diameter of the protein-based particles in the dispersion at pH= 4.2-5.0 was measured by means of a Microtrac (Tradename) Small Particle Analyzer. For this size-analysis 0,5 g of denatured protein product were suspended in 30 ml. ethanol. After sonication for at least 1 h, until all protein is suspended, the measurement was carried out by adding 10 ml ethanolic suspension to the vessel of the Microtrac. Most particles appeared to have under these conditions in Microtrac a diameter in the range from 1 to 9 micrometer (microns), whereas the mean particle size was 3.6 μm.

### Example 2

1 litre of 5% aqueous solution (pH 7.2) of the Bipro (R-Tradename) undenatured whey protein powder (ex. Bio-isolates, PLC, Swansea, UK) was partly denatured by heating 10 minutes at 65°C. Prior to heating lecithin (0.3 wt.%, ex Quest International) was added to the solution.

The obtained colloidal dispersion was cooled to 50°C and acidified to pH 4.8 with 20 wt.% citric acid solution, and concentrated by centrifuge at 3000 rpm after which a spreadable mass, containing about 12 wt.% of mainly alpha-lactalbumin and lecithin, and 88 wt.% of water with undenatured protein was obtained. This edible protein composition had an improved fatty mouthfeel over the protein composition without lecithin.

### Example 3

Experiment 2 was repeated with mono-/diglycerides as added emulsifier prior to heating (Hymono 1103, ex. Quest International). The thus obtained edible plastic protein composition had improved fatty characteristics compared to the protein composition without mono-/diglycerides.

### Example 4

The edible composition was freezed and freeze dried with saccharose as stabilizing agent. The protein composition of example 1 was mixed with saccharose (protein: saccharose = 1:1) prior to freezing and freeze drying. The obtained freeze dried powder (water content 1%) could be reconstituted with water to an edible plastic protein composition with similar fatty mouthfeel as had the original, not freezed and freeze dried protein composition.

Example 5

The edible composition of example 1 was spraydried (nozzle, atomizer) without any additives, and with lactose as stabilizing agent (protein: lactose = 1:1). A spray dried powder (water content 5%) was obtained, which could be reconstituted to an edible protein composition with similar smooth and fatty character as had the original, not spray dried protein composition.

Example 6

A 100 kilogram batch of a mayonnaise-like product was prepared in accordance with the practice of the present invention by admixing the following ingredients:

| | |
|---|---|
| **Edible protein composition** | **45.0 (wt%)** |
| **Vegetable oil** | **35.0** |
| **Sugar** | **5.0** |
| **Food grade acid mixture** | **4.5** |
| **Salt** | **1.5** |
| **Egg yolk** | **3.0** |
| **Stabilizing blend** | **0.35** |
| **Water** | **6.65** |

Example 7

A 100 kilogram fatty-creamy frozen dessert was prepared with a continuous ice-cream apparatus by admixing the following ingredients:

| | |
|---|---|
| **Edible protein composition** | **19.0 (wt%)** |
| **Skim milk powder** | **10.0** |
| **Sugars** | **14.0** |
| **Dextrose** | **4.0** |
| **Stabilizer blend** | **0.1** |
| **Egg yolk** | **1.0** |
| **Water** | **51.9** |

Example 8

A low fat all purpose cream was prepared from the following ingredients:

| | | |
|---|---|---|
| Edible protein composition powder | 2.0 | (wt%) |
| Glyceryl stearate | 5.0 | |
| Polysorbate 80 | 1.0 | |
| Glycerin | 19.0 | |
| Lanolin anhydrous | 1.0 | |
| Liquid paraffin | 18.0 | |
| Avocado oil | 2.0 | |
| Sodium borate | 1.0 | |
| Methyl p-hydroxybenzoate | 0.1 | |
| Propylene glycol | 0.9 | |
| Deionzied water | 49.5 | |
| Perfume composition | 0.5 | |

Example 9

1 litre of 5% aqueous solution (pH 7.2) of the Bipro (R-Tradename) undenatured whey protein powder (ex. Bio-isolates, PLC, Swansea, UK) was partly denatured by heating 10 minutes at 65°C. Prior to heating lecithin (0.3 wt%, ex Quest International) was added to the solution. The heating was conducted in the same way, as described in example 1. The colloidal dispersion obtained was cooled to 50°C and acidified to pH= 4.8 with 20 wt% citric acid solution, and concentrated by centrifuge at 3000 rpm after which a spreadable mass, containing about 12 wt% of mainly alpha-lactalbumin and lecithin, and 88 wt% of water with undenatured protein was obtained. This edible protein composition had an improved fatty mouthfeel over the protein composition without lecithin.

**Claims**

1. Process for the preparation of a food composition, the process comprising successively heating of an aqueous composition at 60-80°C and at a pH of 6.0-7.2 comprising less than 15% by weight of the water, of heat denaturable protein at least partly consisting of whey protein, to at least partly denature said protein, cooling of the composition to a temperature of not less than 45°C, adjusting the pH of the composition to 4.2-5.0 and removing at least part of the remaining undenatured protein present, to provide a composition of at least 5 wt.% of heat denatured protein in the form of microcolloidal particles having a means size in the range of 0.1 to 10 micrometer (microns) at pH= 4.2-5.0, in which the alpha-lactalbumin content and the β-lactoglobulin content, based upon the protein present in the whey are such that the weight ratio of α-lactalbumin to β-lactoglobuline is more than 0.43.

2. Process according to claim 1 wherein the removal of undenatured protein is carried out in such a way that the weight ratio of alpha-lactalbumin to β-lactoglobulin in the composition, based upon the protein present in the whey, is 0.55 - 4.0.

3. Process according to claim 1 or 2 wherein the aqueous denatured protein composition is concentrated to a protein content of at least 7%.

4. Process according to claim 1 wherein the aqueous protein composition is heated for a period from 1 to 30 minutes within the temperature range 60 to 75°C.

5. Process according to any of the preceding claims wherein the aqueous denatured protein containing composition is mixed with carbohydrate and subsequently subjected to freezing and freeze drying.

6. Process according to claim 5 wherein said carbohydrate is a monosaccharide or disaccharide.

7. Process according to claim 1 to 4 wherein the aqueous denatured protein containing composition is spraydried with a nozzle spraydrier or atomizer, optionally mixed with carbohydrate prior to spraydrying.

8. Process according to claim 7 wherein said carbohydrate is a monosaccharide or disaccharide.

9. Process according to claim 5 to 8 wherein carbohydrate is added to the aqueous protein composition in a concentration ratio protein : carbohydrate up to 1:20.

10. Process according to claim 1 wherein an emulsifier a thickener and/or a stabilizer is added to the aqueous protein solution prior to heating in a concentration of up to 5.0 wt.%

11. Process according to claim 10 wherein said emulsifier has a HLB-value less than 10 and is derived from the group of sorbitan esters, mono-/diglycerides, lecithin, or egg yolk.

12. Process according to any of the preceding claims wherein the heat-denaturable protein is a delactosed, demineralised protein, preferably ultrafiltered whey protein.


## Patentansprüche

1. Verfahren zur Herstellung einer Nahrungsmittelzusammensetzung, das umfaßt: das aufeinanderfolgende Erhitzen einer wäßrigen Zusammensetzung, die weniger als 15 %, bezogen auf das Gewicht von Wasser, eines hitzedenaturierbaren Proteins umfaßt, das mindestens teilweise aus Molkeprotein besteht, bei einem pH von 6,0 bis 7,2 auf 60 bis 80°C, um das Protein mindestens teilweise zu denaturieren, das Abkühlen der Zusammensetzung auf eine Temperatur von nicht weniger als 45°C, das Einstellen des pH-Wertes der Zusammensetzung auf 4,2 bis 5,0 und das Entfernen mindestens eines Teils des verbleibenden undenaturiert vorliegenden Proteins, um eine Zusammensetzung von mindestens 5 Gew.-% hitzedenaturiertem Protein in Form mikrokolloidaler Teilchen einer mittleren Größe im Bereich von 0,1 bis 10 Mikrometern (Micron) bei einem pH von 4,2 bis 5,0 zu bilden, in der der alpha-Lactalbumingehalt und der beta-Lactoglobulingehalt, bezogen auf das in der Molke vorliegende Protein, so bemessen sind, daß das Gewichtsverhältnis von alpha-Lactalbumin zu beta-Lactoglobulin mehr als 0,43 beträgt.

2. Verfahren nach Anspruch 2, worin die Entfernung des undenaturierten Proteins in solcher Weise erfolgt, daß das Gewichtsverhältnis von alpha-Lactalbumin zu beta-Lactoglobulin in der Zusammensetzung, bezogen auf das in der Molke vorliegende Protein, 0,55 bis 4,0 beträgt.

3. Verfahren nach Anspruch 1 oder 2, worin die wäßrige denaturierte Proteinzusammensetzung auf einen Proteingehalt von mindestens 7 % konzentriert wird.

4. Verfahren nach Anspruch 1, worin die wäßrige Proteinzusammensetzung für eine Dauer von 1 bis 30 min auf einen Temperaturbereich von 60 bis 75°C erhitzt wird.

5. Verfahren nach irgendeinem der vorhergehenden Ansprüche, worin die denaturiertes Protein enthaltende wäßrige Zusammensetzung mit einem Kohlenhydrat gemischt und dann einem Einfrieren und einer Gefriertrocknung unterzogen wird.

6. Verfahren nach Anspruch 5, worin das Kohlenhydrat ein Mono- oder Disaccharid ist.

7. Verfahren nach Anspruch 1 bis 4, worin die denaturiertes Protein enthaltende wäßrige Zusammensetzung mit einem Düsensprühtrockner oder Zerstäuber sprühgetrocknet und fakultativ vor der Sprühtrocknung mit einem Kohlenhydrat gemischt wird.

8. Verfahren nach Anspruch 7, worin das Kohlenhydrat ein Mono- oder Disaccharid ist.

9. Verfahren nach Anspruch 5 bis 8, worin das Kohlenhydrat der wäßrigen Proteinzusammensetzung in einem Konzentrationsverhältnis Protein:Kohlenhydrat von bis zu 1:20 zugefügt wird.

10. Verfahren nach Anspruch 1, worin der wäßrigen Proteinlösung vor dem Erhitzen ein Emulgator, ein Verdickungsmittel und/oder ein Stabilisator in einer Konzentration von bis zu 5,0 Gew.-% zugefügt wird/werden.

**11.** Verfahren nach Anspruch 10, worin der Emulgator einen HLB-Wert unter 10 hat und von der Gruppe von Sorbitanestern, Mono-/Diglyceriden, Lecithin oder Eigelb abgeleitet ist.

**12.** Verfahren nach irgendeinem der vorhergehenden Ansprüche, worin das hitzedenaturierbare Protein ein entlactosiertes, entmineralisiertes Protein, vorzugsweise ultrafiltriertes Molkeprotein, ist.

**Revendications**

**1.** Procédé de préparation d'une composition alimentaire, qui consiste :
à chauffer successivement une composition aqueuse à 60-80°C et à pH 6,0-7,2 comprenant moins de 15% en poids d'eau, d'une protéine dénaturable par la chaleur consistant au moins partiellement en protéine de petit lait, pour dénaturer au moins partiellement ladite protéine, à refroidir la composition à une température qui n'est pas inférieure à 45°C ; à régler le pH de la composition à une valeur de 4,2 à 5,0 et à soutirer au moins une partie de la protéine non dénaturée restante, afin de fournir une composition d'au moins 5% en poids de protéine dénaturée par la chaleur sous forme de particules microcolloïdales d'une granulométrie moyenne de 0,1 à 10 μm, à pH 4,2 - 5,0, la teneur en alpha-lactalbumine et la teneur en bêta-lactaglobuline par rapport à la protéine présente dans le petit lait étant telles que le rapport pondéral de l'alpha-lactalbumine à la bêta-lactoglobuline est supérieur à 0,43.

**2.** Procédé selon la revendication 1, dans lequel on effectue le soutirage de la protéine non dénaturée de façon telle que le rapport pondéral de l'alpha-lactalbumine à la bêta-lactoglobuline dans la composition, par rapport à la protéine présente dans le petit lait, est de 0,55 à 4,0.

**3.** Procédé selon la revendication 1 ou 2, dans lequel on concentre la composition protéinée dénaturée aqueuse jusqu'à une teneur en protéine d'au moins 7%.

**4.** Procédé selon la revendication 1, dans lequel on chauffe la composition aqueuse des protéines pendant une durée de 1 à 30 minutes, à une température comprise entre 60 et 75°C.

**5.** Procédé selon l'une quelconque des revendications précédentes , dans lequel on mélange la composition contenant la protéine aqueuse dénaturée avec de l'hydrate de carbone et, ensuite, on soumet à la congélation et à la lyophilisation.

**6.** Procédé selon la revendication 5, dans lequel ledit hydrate de carbone est un monosaccharide ou disaccharide.

**7.** Procédé selon l'une quelconque des revendications 1 à 4, dans lequel on sèche par pulvérisation la composition aqueuse contenant la protéine dénaturée avec un séchoir à pulvérisation avec ajutage ou atomiseur, facultativement en mélange avec l'hydrate de carbone avant le séchage par pulvérisation.

**8.** Procédé selon la revendication 7, dans lequel ledit hydrate de carbone est un mono-saccharide ou un disaccharide.

**9.** Procédé selon l'une quelconque des revendications 5 à 8, dans lequel on ajoute l'hydrate de carbone à la composition de protéine aqueuse en un rapport de concentration de protéine à l'hydrate de carbone allant jusqu'à 1:20.

**10.** Procédé selon la revendication 2, dans lequel on ajoute un émulsifiant, un épaississant et/ou un stabilisant à la solution protéinée aqueuse avant chauffage en une concentration pouvant aller jusqu'à 5,0% en poids.

**11.** Procédé selon la revendication 10, dans lequel ledit émulsifiant présente un indice d'amphipathie inférieur à 10 et il est dérivé des esters de sorbitanne, des mono- ou di-glycérides, de la lécithine ou du jaune d'oeuf.

**12.** Procédé selon l'une quelconque des revendications précédentes, dans lequel la protéine dénaturable par la chaleur est une protéine déminéralisée et délactosée, de préférence une protéine de petit lait ultrafiltrée.